(19) Europäisches Patentamt / European Patent Office / Office européen des brevets

(11) **EP 3 439 532 B1**

(12) **EUROPÄISCHE PATENTSCHRIFT**

(45) Veröffentlichungstag und Bekanntmachung des Hinweises auf die Patenterteilung:
**03.11.2021 Patentblatt 2021/44**

(51) Int Cl.:
***A61B 3/00*** *(2006.01)*     ***A61F 2/16*** *(2006.01)*

(21) Anmeldenummer: **17715678.3**

(22) Anmeldetag: **03.04.2017**

(86) Internationale Anmeldenummer:
**PCT/EP2017/057852**

(87) Internationale Veröffentlichungsnummer:
**WO 2017/174508 (12.10.2017 Gazette 2017/41)**

(54) **VERFAHREN ZUR UNTERSTÜTZUNG DER AUSWAHL EINER IN EIN AUGE ZU IMPLANTIERENDEN IOL**

METHOD FOR SUPPORTING THE SELECTION OF AN IOL TO BE IMPLANTED IN AN EYE

PROCÉDÉ D'AIDE À LA SÉLECTION D'UNE LENTILLE INTRAOCULAIRE À IMPLANTER DANS UN OEIL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.04.2016 DE 102016205677**

(43) Veröffentlichungstag der Anmeldung:
**13.02.2019 Patentblatt 2019/07**

(73) Patentinhaber: **Carl Zeiss Meditec AG**
**07745 Jena (DE)**

(72) Erfinder:
• **BÜHREN, Tobias**
**89073 Ulm (DE)**
• **CHEUNG, Wai-Yin**
**07749 Jena (DE)**

(74) Vertreter: **Kintzel, Klaus-Peter**
**Carl Zeiss AG**
**Patentabteilung**
**Carl-Zeiss-Promenade 10**
**07745 Jena (DE)**

(56) Entgegenhaltungen:
WO-A1-2010/028654     WO-A1-2012/163980
WO-A2-2010/064150     DE-A1-102011 113 953
DE-A1-102013 020 706

**Beschreibung**

Die vorliegende Erfindung betrifft ein Verfahren zur Unterstützung der präoperativen Auswahl einer, in ein Auge zu implantierenden, intraokularen Linse, um die Ergebnisse refraktiver Eingriffe am Auge zu optimieren.

Nach dem bekannten Stand der Technik werden IOLs auf Basis gemessener und/oder geschätzter Größen ausgewählt bzw. angepasst, wobei nur einzelne Parameter in Form von Einzelmesswerten oder als ein Mittelwert über definierte Patientengruppen berücksichtigt werden.

DE 10 2011 113953 offenbart ein Verfahren zur automatisierten Optimierung der Berechnung einer zu implantierenden IOL, wobei die mittlere Abweichung der Hornhauttomographie in das Berechnungsmodell für das Raytracing für zukünftige Berechnung der zu implantierenden IOL einfließt.

Bei den heute üblichen, Formel-basierten Verfahren erfolgt die Auswahl bzw. Anpassung der optimalen, intraokularen Linse (IOL) ausschließlich nach deren Merkmalen, wie beispielsweise Typ, Brechkraft, Asphärizität und Multifokalität. Bei den Formel-basierten Verfahren erfolgt die Vorhersage einer postoperativen Linsenposition anhand unterschiedlicher Annahmen, die auf den verschiedensten biometrischen Parametern des Auges basieren. Es wird lediglich ein virtueller Rechenparameter vorhergesagt, der zwar konzeptionell von einer IOL-Lage abgeleitet, aber durch Konstantenoptimierung erzeugt wurde. Die dadurch bestimmte, formelspezifische Linsenposition wird auch als effektive Linsenposition (ELP) bezeichnet und ist nicht mit einer tatsächlichen, postoperativen Linsenposition zu verwechseln.

Die Auswahl der geeigneten Intraokularlinse (IOL) für einen Patienten obliegt dem Kataraktchirurgen. Dabei muss der Chirurg viele Faktoren beachten. Zum einen sollte in Abhängigkeit der individuellen biometrischen Parameter des Auges die geeignete Berechnungsmethode des IOL-Brechwerts gewählt werden. Hierzu werden in der Regel bei außergewöhnlich langen, normalen oder außergewöhnlich kurzen Augen, verschiedene mehr oder minder geeignete Formeln zur Berechnung verwendet. Deren Input-Parameter basieren im einfachsten Fall auf Keratometrie und Achslänge des Auges, wobei die Formeln meist, aufgrund ihrer vereinfachten Modellannahmen, zusätzlich einen empirisch ermittelten Korrekturfaktor, wie beispielsweise die sogenannte A-Konstante enthalten.

Das zurzeit am weitesten verbreitete Berechnungsmethode sind sogenannte IOL-Formeln, z. B. nach Holladay, Hoffer, Binkhorst, Colenbrander, Shammas, oder SRK. Danach berechnet sich die Refraktion D (Ausgangs-Bewertungsparameter) des Patienten nach Einsetzen der IOL zu:

$$D = DIOL - f(K, AL, VKT, A) \qquad (1)$$

wobei f() eine klassisch bekannte IOL-Formel und
DIOL die Brechkraft der IOL,
K der gemessene Keratometriewert,
AL die gemessene Achslänge des Auges,
VKT die gemessene Vorderkammertiefe und
A eine IOL-Typ-abhängige konstante Eingangsgrößen sind.

Zur Auswahl der IOL gibt sich der Arzt eine Zielrefraktion vor (D = DZIEL). Eine Optimierung erfolgt, indem der Arzt DIOL und A für verschiedene IOLs variiert und die Refraktion gemäß (1) berechnet. In vielen Fällen verwendet der Arzt IOLs des gleichen Typs, so dass keine Variation in A erfolgt und die Optimierung auf eine Formelberechung gemäß DIOL = DZIEL + f(K, AL, VKT, A) hinaus läuft.

Die Konstante A in den Formeln wird empirisch über ein Patientenensemble ermittelt, um die Formelwerte an die real sich ergebenden optimalen Refraktionswerte anzupassen. Diese Anpassung stellt jedoch nur sicher, dass der Mittelwert der Refraktionswerte über das Testensemble mit der Formel übereinstimmt.

Statistische Fehler der IOL-Formel werden vom Arzt typischerweise dadurch berücksichtigt, dass er aus seiner Erfahrung weiß, dass seine real erzielten Refraktionswerte über seine Patienten eine gewisse Schwankung um die Zielrefraktion haben. Will er deren Einfluss minimieren so hält er eine Korrektur der Zielrefraktion vor. Beispielsweise hat ein Arzt bei Patienten mit myopen Augen typische Abweichungen von +/- 0,25D gegenüber Zielrefraktion, dann wird er auf eine Refraktion von -0,25D zielen, um mit hoher Wahrscheinlichkeit zu vermeiden, dass das Auge der Patienten intolerabel hyperop wird, Diese Methode stellt im Mittel über das Patienten-Ensemble eine gute Strategie dar.

Allerdings könnte die typische Schwankung um die Zielrefraktion bzw. der Vorhalt verringert werden, wenn als Ausgangsgröße statt eines Mittelwertes über ein Patientenensemble individuelle Eingangsparameter des einzelnen Patienten verwendet werden würden.

Um systematische Fehler zu minimieren, werden derzeit nach dem Stand der Technik verschiedene Ansätze gewählt.

So verwendet eine Reihe von Ärzten für jede ethnische Gruppe ihrer Patienten eine verschiedene A-Konstante. Dadurch lassen sich die systematischen Fehler reduzieren und, sofern die statistische Streuung in der jeweiligen Gruppe geringer ist, auch die statistischen Fehler.

Andere Ärzte verwenden in Abhängigkeit von definierten Ausgangsbedingungen, wie beispielsweise Patienten mit langen Achslängen oder mit vorheriger refraktiver Hornhautchirurgie, unterschiedliche Biometrieformeln, die an die jeweiligen Bedingen besser angepasst sind, oder die die Messung zusätzlicher Parameter, wie Vorderkammertiefe oder Linsendicke, voraussetzen. Auch hier werden insbesondere die systematischen Fehler verringert, wobei allerdings teilweise aufgrund der zusätzlichen gemessenen Parameter die statistischen Fehler zunehmen können.

So wird beispielsweise in der US 5,968,095 A ein Verfahren zum präoperativen Auswählen einer intraokularen Linse beschrieben, bei dem gewährleistet werden soll, dass das Auge eine gewünschte postoperative Brechkraft aufweist. Dies soll dadurch erreicht werden dass der Ort der haptischen Linsenebene, die korneale Brechkraft und die axiale Länge des Auges bestimmt und die wünschte postoperative Brechkraft ausgewählt wird. Für eine zu implantierende IOL, deren Brechkraft und Geometrie bekannt sind wird ein Versatz zwischen der haptischen Ebene der Linse und dem vorderen Vertex vorgegeben, so als wäre sie in ihrem implantierten Zustand. Im Anschluss wird durch Berechnung überprüft, ob der Fokus der ausgewählten IOL, mit den zuvor genannten Vorgaben sowie den Brechungsindices der okularen Flüssigkeiten, postoperativ auf die Retina des Auges fällt. Ist dies nicht der Fall, so wird die Berechnung für eine andere IOL, mit einer anderen Brechkraft und/oder Geometrie, erneut durchgeführt. Für die Implantation wird eine zur Verfügung stehenden IOL mit der nächstkommenden Brechkraft ausgewählt, für die eine Fokussierung auf die Retina berechnet worden ist.

Nachteilig wirkt sich aus, dass die Formel-basierten Verfahren insbesondere bei der Berechnung torischer IOLs nicht die erforderliche Qualität erreichen und die Auswahl des richtigen IOL-Design für den jeweiligen bestimmten Patienten extrem erschweren. Dabei liefern diese Verfahren nicht nur suboptimale Ergebnisse sondern mitunter auch überraschende Fehlberechnungen. Dieser Nachteil lässt sich auch dadurch nicht vollständig beheben, dass keratometrische Messungen der Hornhauttopographie mit verschiedenen Geräten erfolgen und die Messwerte verglichen werden, um Asymmetrien der Hornhaut auszuschließen.

Für einen Kataraktchirurgen wäre es deshalb von Vorteil, wenn weitere Informationen über die vorausgesagte visuelle Leistung und Eignung der berechneten IOLs zur Verfügung stünden.

Eine alternative, aber noch nicht sehr weit verbreitete Methode, ist das Raytracing. Unter Raytracing ist, wie schon der Begriff andeutet ("ray" = Strahl und "to trace" = zurückverfolgen) ein Verfahren zur Strahlverfolgung zu verstehen. Gegenstände in unserer Umwelt nehmen wir bekanntlich nur wahr, weil sie von einer Lichtquelle bestrahlt werden und sie diese Lichtstrahlen reflektieren, von denen ein Teil schließlich unsere Augen erreichen. Das Raytracing-Verfahren simuliert dieses elementare Naturphänomen. Ist das optische System, d. h. das individuelle menschliche Auge mit all seinen optischen Elementen bekannt, so kann mittels des Raytracing ein "real" auf der Retina entstehendes Abbild berechnet werden. Das Verfahren beruht somit auf einem detaillierten Augenmodel unter Verwendung der Hornhauttopographie des Auges. Bei dieser Methode werden keine allgemeinen Korrekturfaktoren (A-Konstanten) verwendet, jedoch sind gewisse Annahmen was die effektive (postoperative) Linsenposition (ELP) angeht erforderlich. Diese Methode eignet sich für Augen mit den unterschiedlichsten biometrischen Parametern, wie beispielsweise: lang, normal, kurz, post-LASIK, usw.

Mit Hilfe von Raytracing werden dann der IOL-Brechwert und die Restrefraktion berechnet. Um dabei eine gute Korrelation mit der subjektiven Sehschärfe, d. h. ein mit der Wahrnehmung des Patienten vergleichbares Ergebnis zu erzielen, können verschiedene Auswahlkriterien bzw. Metriken zur Berechnung herangezogen werden.

Obwohl das Raytracing bereits seit mehr als einem Jahrzehnt für Simulationen am Auge bekannt ist, hat es sich für die Berechnung der Brechkraft von IOLs bisher nicht durchsetzen können. Einer der Gründe dafür könnte sein, dass die Modellierung eines Auges sehr komplex ist, da es eine Vielzahl von Parametern zu berücksichtigen gilt.

Der Vorteil des Raytracing liegt unter anderem darin begründet, dass Informationen zum Design der IOLs bei den Berechnungen berücksichtigt werden. Allerdings scheint die Robustheit der einfacheren, Formel-basierten Verfahren diese Vorteile zu mindern.

Wünschenswert wäre daher die beim Raytracing anhand eines Augenmodelles stattfindende Simulationen der Sehleistung mit neuesten, robusten IOL-Formeln zu kombinieren. Die dabei verwendeten unterschiedlichen Ansätze bezüglich der Vorhersage der IOL-Position scheint eine derartige Kombination aber zu verhindern.

Mit den Formel-basierten Verfahren wird eine effektive Linsenposition (ELP) vorhergesagt, die hinsichtlich auftretender Fehler kompensieren ist und dadurch robuste Ergebnisse liefert. Im Gegensatz basiert Raytracing in der Regel auf der (tatsächlichen) physischen Linsenposition (PLP), die nichts mit der ELP zu tun hat und deshalb auch in keiner mathematisch zu beschreibenden Beziehung zu dieser steht. Es ist daher auch nicht möglich aus einer effektiven Linsenposition (ELP) die physische Linsenposition (PLP) abzuleiten.

Der vorliegenden Erfindung liegt die Aufgabe zugrunde ein Verfahren zur Unterstützung der Auswahl einer in ein Auge zu implantierenden IOL zu entwickeln, bei dem die unter Verwendung einer bekannten Berechnungsformeln bestimmten IOLs einer zusätzlichen Bewertung unterzogen werden, damit die Entscheidung zur Auswahl einer IOL für den Arzt vereinfacht wird. Dabei soll es dem Arzt möglich sein, die Ergebnisse der Berechnung zu überprüfen und geeignet erscheinende IOLs miteinander zu vergleichen.

Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Insbesondere wird diese Aufgabe durch das Verfahren zur Unterstützung der Auswahl einer in ein Auge zu implantierenden IOL, basierend auf der Verwendung einer der bekannten Berechnungsformeln, mit der anhand der geometrischen Daten des Auges die zu implantierende IOL und deren effektiven Linsenpositionen (ELP) ermittelt wird, dadurch gelöst, dass die ermittelte IOL in ein individuelles Modell des zu implantierenden Auges an der effektiven Linsenposition (ELP) eingefügt, so lange verschoben bis die Restrefraktion des individuellen Modells der mit der bekannten Berechnungsformel ermittelten Restrefraktion entspricht und mittels Raytracing die zu erwartenden Sehleistungen simuliert wird.

Obwohl das vorgeschlagene Verfahren zur Unterstützung der generellen Auswahl einer in ein Auge zu implantierenden IOL geeignet ist, liegen dessen Vorzüge bei der Auswahl torischer IOL bzw. bei der Auswahl einer IOL für anormale Augen.

Die Erfindung wird nachfolgend anhand von Ausführungsbeispielen näher beschrieben.

Bei dem erfindungsgemäßen Verfahren zur Unterstützung der Auswahl einer in ein Auge zu implantierenden IOL, basierend auf der Verwendung einer der bekannten Berechnungsformeln, werden anhand der geometrischen Daten des Auges die zu implantierende IOL und deren effektiven Linsenpositionen (ELP) ermittelt, in ein individuelles Modell des zu implantierenden Auges an der effektiven Linsenposition (ELP) eingefügt, so lange verschoben bis die Restrefraktion des individuellen Modells der mit der bekannten Berechnungsformel ermittelten Restrefraktion entspricht und mittels Raytracing die zu erwartenden Sehleistungen simuliert.

Erfindungsgemäß berücksichtigt das individuelle Augenmodell neben der Vorderkammertiefe und der Augenlänge die geometrischen Eigenschaften und optischen Wirkungen der Hornhaut. Insbesondere wird das individualisierte Augenmodell verwendet, um aus der effektiven Linsenposition (ELP) für eine IOL, deren physische Linsenposition (PLP) zu generieren.

Die physische Linsenposition (PLP) basiert hierbei in abgewandelter Form lediglich auf den geometrischen Daten des Auges, ohne die optischen Parameter der berechneten IOL zu berücksichtigen, da diese in der Regel nicht vorhanden sind. Bei dem abgewandelten Augenmodell für das Raytracing werden aber insbesondere die optischen Daten der Hornhaut berücksichtigt. Asymmetrien der Hornhaut sind eine wesentliche Fehlerquelle für die Formel-basierten Verfahren.

Eine reale Simulation wäre nur möglich, wenn die Designdaten der entsprechenden IOLs vorhanden wären. Diese werden jedoch von den Herstellern der IOL in der Regel nicht veröffentlicht. Somit ist die hier vorgeschlagene Simulation lediglich als generisch zu betrachten.

Bei dem vorgeschlagenen Verfahren wird für die Simulation die Lage der ermittelten IOLs innerhalb des individuellen Modell des zu implantierenden Auges so lange verschoben, bis die Restrefraktion des individuellen Modells der mit der bekannten Berechnungsformel ermittelten Restrefraktion entspricht.

Durch die Simulation der Sehleistung unter Verwendung des individuellen Augenmodells können mit den Berechnungsformeln bestimmten Refraktionen kontrolliert werden.

Erfindungsgemäß kann mit dem Verfahren durch das Simulieren der zu erwartenden Sehleistungen bei äquivalenter Restrefraktion eine Plausibilitätskontrolle der mit der Berechnungsformel bestimmten IOL und deren ermittelten effektiven Linsenposition (ELP) erfolgen.

Somit kann die Eignung der Formel-basiert ermittelten IOLs bewertet werden, wobei ausgehend von der berechneten ELP eine Vorhersage der PLP erfolgen kann. Insbesondere sind dadurch Fälle erkennbar, in denen ein falscher Formelansatz verwendet wurde. Dies wäre beispielsweise der Fall, wenn die Hoffer-Formel für lange Augen Verwendung findet. Ein solcher Fall würde durch das Raytracing im individuellen Augenmodell erkannt werden.

In derartigen Fällen würde sich die abgeleitete ELP im Ergebnis des Raytracing an einer unrealistischen Position, beispielsweise außerhalb des Kapselsackes befinden.

Das vorgeschlagene Verfahren bietet weiterhin die Möglichkeit, IOLs mit verschiedenen Designs in das individuelle Modell des zu implantierenden Auges einzufügen, die zu erwartenden Sehleistungen bei äquivalenter Restrefraktion mittels Raytracing zu simulieren, die Ergebnisse zu vergleichen und die IOL mit dem am besten geeigneten Design auszuwählen.

Mit der erfindungsgemäßen Lösung wird ein Verfahren zur Auswahl einer in ein Auge zu implantierenden IOL zur Verfügung gestellt, mit dem die Entscheidung zur Auswahl einer IOL für den Arzt unterstützt und dadurch vereinfacht wird.

Die vorgeschlagene Lösung verbindet hierbei die Robustheit Formel-basierter Berechnungsansätze mit der visuellen Performance-Simulation des Raytracing in einem individuellen Augenmodell.

Im weitesten Sinne kann das vorgeschlagene Verfahren als Bildsimulation der Berechnungsformel betrachtet werden.

**Patentansprüche**

1. Verfahren zur Unterstützung der Auswahl einer in ein Auge zu implantierenden IOL, basierend auf der Verwendung einer Berechnungsformel, mit der anhand der geometrischen Daten des Auges die zu implantierende IOL und deren effektiven Linsenpositionen (ELP) ermittelt wird, wobei die ermittelte IOL in ein individuelles Modell des zu implantierenden Auges an der effektiven Linsenposition (ELP) eingefügt, so lange verschoben bis die Restrefraktion des individuellen Modells der mit der Berechnungsformel ermittelten Restrefraktion entspricht und mittels Raytracing die zu erwartenden Sehleistungen simuliert wird.

2. Verfahren nach Anspruch 1, **dadurch gekennzeichnet, dass** das individuelle Augenmodell neben der Vorderkammertiefe und der Augenlänge die geometrischen Eigenschaften und optischen Wirkungen der Hornhaut berücksichtigt.

3. Verfahren nach den Ansprüchen 1 und 2, **dadurch gekennzeichnet, dass** durch das Simulieren der zu erwartenden Sehleistungen bei äquivalenter Restrefraktion eine Plausibilitätskontrolle der mit der Berechnungsformel bestimmten IOL und deren ermittelten effektiven Linsenposition (ELP) erfolgt.

4. Verfahren nach Anspruch 1, **dadurch gekennzeichnet, dass** IOLs mit verschiedenen Designs in das individuelle Modell des zu implantierenden Auges eingefügt, die zu erwartenden Sehleistungen bei äquivalenter Restrefraktion mittels Raytracing simuliert und die IOL mit dem am besten geeigneten Design ausgewählt wird.

**Claims**

1. Method for assisting the selection of an IOL to be implanted into an eye, on the basis of the use of a calculation formula with which the IOL to be implanted and the effective lens positions (ELP) thereof are ascertained on the basis of the geometric data of the eye, wherein the ascertained IOL is inserted into an individual model of the eye to be implanted at the effective lens position (ELP) and displaced until the residual refraction of the individual model corresponds to the residual refraction ascertained using the calculation formula, and the expected visual performance is simulated by means of ray tracing.

2. Method according to Claim 1, **characterized in that** the individual eye model takes account of the geometric properties and optical powers of the cornea in addition to the anterior chamber depth and the eye length.

3. Method according to Claims 1 and 2, **characterized in that** simulating the expected visual performances at an equivalent residual refraction implements a plausibility check for the IOL determined by the calculation formula and the ascertained effective lens position (ELP) thereof.

4. Method according to Claim 1, **characterized in that** IOLs with different designs are inserted into the individual model of the eye to be implanted, the expected visual performances at equivalent residual refraction are simulated by means of ray tracing and the IOL with the most suitable design is selected.

**Revendications**

1. Procédé d'aide à la sélection d'une lentille intraoculaire (IOL) à implanter dans un œil, sur la base de l'utilisation d'une formule de calcul par laquelle, à l'aide des données géométriques de l'œil, l'IOL à implanter et ses positions de lentille efficaces (ELP) sont déterminées,
dans lequel l'IOL déterminée est insérée dans un modèle individuel de l'œil récepteur à la position de lentille efficace (ELP) en étant déplacée jusqu'à ce que la réfraction résiduelle du modèle individuel corresponde à la réfraction résiduelle déterminée par la formule de calcul, et les performances visuelles attendues sont simulées au moyen d'un lancer de rayons.

2. Procédé selon la revendication 1, **caractérisé en ce que** le modèle d'œil individuel prend en compte les propriétés géométriques et les effets optiques de la cornée en plus de la profondeur de chambre antérieure et de la longueur d'œil.

3. Procédé selon les revendications 1 et 2, **caractérisé en ce que** la simulation des performances visuelles attendues pour une réfraction résiduelle équivalente permet d'effectuer un contrôle de plausibilité de l'IOL définie par la formule de calcul et de sa position de lentille efficace (ELP) déterminée.

4. Procédé selon la revendication 1, **caractérisé en ce que** des IOL de différentes conceptions sont insérées dans le modèle individuel de l'œil récepteur, les performances visuelles pour une réfraction résiduelle équivalente sont simulées au moyen d'un lancer de rayons, et l'IOL ayant la conception la mieux appropriée est sélectionnée.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011113953
- US 5968095 A